# EUROPEAN PATENT APPLICATION

(11) **EP 1 046 706 A1**
(43) Date of publication of application: **25.10.2000**
(21) Application number: 99107354.5
(22) Date of filing: 21.04.1999
(51) Int. Cl.: C12M 1/40, C12C 11/09

(54) **Method and apparatus for the continuous biocatalytic conversion of aqueous solutions, having one or more degassing stages**

(71) Applicant: GEA Liquid Processing Scandanavia A/S, 8600 Silkeborg (DK)
(72) Inventor: Andersen, Kjeld, DK-8600 Silkeborg (DK); Lommi, Heikki Olavi, FIN-02640 Kantvik (FI); Viljava, Timo Tapio, FIN-02460 Kantvik (FI); Pajunen, Esko Juhani, FIN-00250 Helsinki (FI); Lynch, Francis J., Glenageary County Dublin (IE); Bergin, Joseph, Leixlip County Kildare (IE)
(74) Representative: Heselberger, Johannes

(57) **Abstract**

The invention relates to a method and an apparatus for the continuous biocatalytic conversion of aqueous solutions containing biocatalytically convertible material in a reactor, having one or more converting/degassing stages in parallel or serial interconnection, each converting/degassing stage comprising a bioreactor and a degassing device in serial interconnection, where in each converting/degassing stage in a first process step an aqueous solution containing biocatalytically convertible material is fed in continuous flow to the inlet of the bioreactor where it is contacted with a biocatalyst, and where in a second process step the biocatalytically converted solution is transferred from an outlet of the bioreactor to an inlet of the degassing device, and at least part of the degassed solution leaving the outlet of the last degassing device in line is fed to the inlet of the first bioreactor in line in a continuous flow. The invention further relates to an apparatus for carrying out such a method.

## Description

The invention relates to a method and an apparatus for the continuous biocatalytic conversion of aqueous solutions containing biocatalytically convertible material in a reactor, having one or more converting/degassing stages in parallel or serial interconnection, each converting/degassing stage comprising at least one bioreactor and at least one degassing device in serial interconnection, where in each converting/degassing stage in a first process step an aqueous solution containing biocatalytically convertible material is fed in continuous flow to the inlet of a first bioreactor or to the common inlet of two or more first bioreactors in parallel interconnection where it is contacted with at least one biocatalyst under formation of at least one gas and at least one solid or liquid product, and in a second process step the biocatalytically converted solution is transferred from an outlet of the last bioreactor or a common outlet line of two or more last bioreactors in parallel interconnection to an inlet of the degassing device, and at least part of the biocatalytically converted, degassed solution leaving the outlet of the last degassing device in line is fed to the inlet of the first bioreactor in line in a continuous flow. The invention further relates to an apparatus for carrying out such a method.

Interest in applications utilizing immobilized biocatalysts, like enzymes and microbes, has increased during recent years. Continuous biocatalytic conversion with immobilized biocatalysts would cut production costs considerably for example by reducing conversion time and the amount of waste associated with spent biomass. An especially interesting field of biocatalytic conversion is the production of non-alcoholic and alcoholic beverages, e.g. in the brewing and wine industry, or the production of gases, like methane, by biocatalytic conversion of organic matter.

Immobilized yeast technology is used today successfully in full industrial scale by several breweries for the manufacture of alcohol-free beer or for the secondary fermentation (maturation) of primary fermented beer. In both processes the yeast is immobilized on a fixed bed of solid, incompressible carrier material in a column reactor, through which wort or green beer is continuously passed as a plug flow. This is relatively easy to accomplish because in the manufacture of alcohol-free beer the wort is not fermented, and in secondary fermentation the green beer is almost fully attenuated so that the small amount of carbon dioxide formed will stay in solution.

In primary fermentation of wort the situation is much more difficult. The amount of carbon dioxide gas released during fermentation (biocatalytic conversion) of the wort is much larger than in the processes mentioned above and will cause channelling of the carrier bed, disturb the plug flow, reduce the effective reactor volume and cause foaming of the product. Another problem is the removal of heat of reaction generated in a rapid biocatalytic conversion. Thus the maintenance of reactor temperature is difficult if not impossible. This is a major drawback in the fermentation of beverages where temperature has a significant effect on the yeast metabolism and on the development of flavour compounds.

During the years, numerous laboratory and pilot scale studies on immobilized primary fermentation of beer have been published, see e.g. Monograph XXIV of European Brewery Convention, *Symposium Immobilized Yeast Applications in the Brewing Industry,* Verlag Hans Carl Getränke-Fachverlag, Nümberg, 1996, ISBN 3-418-00749-X. For example, Yamauchi and Kashihara have used a two-stage process comprising a continuously stirred tank and a packed bed reactor in series. Kronlöf et al. have used two packed bed reactors in series. In these and other cases, however, technical problems, have made practical operation of the processes difficult leading to poor control of biocatalytic conversion. As described above, an essential cause of these technical problems is the vigorous formation of carbon dioxide. Furthermore, the large amount of heat of reaction generated during primary fermentation makes temperature control difficult, if not impossible to achieve. Both Yamauchi and Kashihara and Kronlöf et al. have reached fermentation times of about two days.

The US-A 5,079,011 discloses a method for producing ethanol and alcoholic beverages in which a liquid containing biocatalytically convertible matter is pumped through a series of interconnecting reactors with immobilized yeast. In the interconnection points, pressure is released to remove most of the carbon dioxide formed in the preceding reactor. Thus, high ethanol concentrations may be achieved without problems caused by gaseous carbon dioxide. This method is, however, expensive and impractical because several reactors and pressure release devices must be used to reach even moderate ethanol concentrations. The pressure is limited by the reactor design as well as by the pressure tolerance of the yeast. As an example, in a beer fermentation with a target ethanol concentration of 5,5 % w/w, in which the maximum allowable pressure in one reactor is 4 bar and the fermentation temperature is 15 °C, the solubility of carbon dioxide is 7,0 g/l. In a pressure of 1 bar the solubility is, respectively, 1,8 g/l. Hence, the maximum amount of carbon dioxide allowed to form in each reactor is 5,2 g/l, which corresponds to an ethanol concentration of 0,54 %. Thus, eleven reactors would be needed for this process. In practice, the number of reactors required would be much higher.

The above mentioned problems apply in principle to all other biocatalytical processes, where an aqueous solution containing biocatalytically convertible material is converted under the formation of at least one gas and at least one product by the use of a biocatalyst. Examples for such processes are the biocatalytical production of pharmaceuticals or the biocatalytical treatment of product streams, where the aim is the production of one or more usable gases, e.g., methane.

It was thus an object of the invention to provide a process which overcomes the above mentioned shortcomings. It was especially an object of the invention to provide a process which allows for the continuous biocatalytic conversion of aqueous solutions containing biocatalytically convertible materials. It was a further object of the invention to provide a process for such a biocatalytic conversion which is faster than prior art processes or utilizes less equipment, or both. It was still a further object of the invention, to provide a process for such a biocatalytic conversion which has a better overall controllability, a controlled steady state, the economical advantage of having a compact system, insensitivity to contamination due to a high level of metabolic products, a more constant nutrient flow compared to processes known from the prior art and a better flow control.

It was a further object of the invention to provide an apparatus for successfully carrying out the described process.

The objects of the invention are solved by circulating the liquid to be converted through a reaction loop in one or more cycles, where gases produced during the biocatalytic conversion are removed during each cycle after the biocatalytic conversion.

If a recycle process like the one described in the present invention, is used in the production of alcohol, e.g. in the production of alcoholic beverages, the biocatalytic conversion takes place close to the end concentration of products. It is known that the rate of ethanol fermentation is limited due to product inhibition. Therefore, one would expect that the biocatalytic conversion in this case would be slower than in a once-through process without recycle. The inventors have, however, found out that the biocatalytic conversion is very fast. Using the present invention in the fermentation of beer, high gravity beer can be fermented in 20 hours or less.

Subject of the invention is thus a method for the continuous biocatalytic conversion of aqueous solutions containing biocatalytically convertible material in a reactor having one or more converting/degassing stages in parallel or serial interconnection, each converting/degassing stage comprising a bioreactor or two or more bioreactors and a degassing device or two or more degassing devices, where the bioreactor or the bioreactors and the degassing device or the degassing devices are in serial interconnection, and where in each converting/degassing stage in a first process step an aqueous solution containing biocatalytically convertible material is fed to the inlet of the bioreactor where it is contacted with a biocatalyst under formation of a biocatalytically converted solution containing at least one gas and at least one product, and where in a second process step the biocatalytically converted solution is transferred from an outlet of the bioreactor or a common outlet line of two or more bioreactors to an inlet of the degassing device or a common inlet line to two or more degassing devices, where it is at least partially degassed to form a degassed solution and at least part of the degassed solution leaving the outlet of the last degassing device or a common outlet of two or more last degassing devices in line, is fed to the inlet of the first bioreactor in line or to a common feeding line for the inlets of two or more first bioreactors, in a continuous flow.

A further subject of the invention is an apparatus for the continuous biocatalytic conversion of aqueous solutions containing biocatalytically convertible material comprising:
- a bioreactor or two or more bioreactors loaded with at least one biocatalyst, each bioreactor having at least one inlet and at least one outlet,
- a circuit line having an entrance connected with at least one outlet of at least one bioreactor via at least one device for degassing the aqueous solution, and at least one exit connected with at least one inlet of at least one bioreactor via a device for circulating the aqueous solution,
- at least one feeding line connected to the circuit line at a location downstream of the device for circulating the aqueous solution, and
- at least one outlet (withdrawal line) connected to the circuit line at a location upstream of the device (4) for circulating the aqueous solution.

The term "gas", as used in the present context, relates to matter which is in the gaseous state at a temperature of about 0 °C and a pressure of about 1 bar. The term "gas" thus also encompasses such matter, which is partially or completely soluble in the aqueous solution or is in the liquid state under the conditions in the bioreactor, but would, under the circumstances mentioned above, be in the gaseous state. An example for a gas which is soluble in the above mentioned aqueous solution at least to a high extent, is carbon dioxide.

The term "product" or "products", as used in the present context, relates to solid or liquid metabolic products as produced by the employed biocatalyst, irrespective if the gas or such products determine the economical value of the process according to the invention. In a preferred embodiment, the products are at least partially soluble in the aqueous solution containing the biocatalytically convertable matter. The metabolism of the biocatalyst can result in only one product, it is, however, also within the scope of the present invention that the biocatalyst produces two or more products, at least one of which is a desired product. Examples for desired products are ethanol, e.g. in wine, beer or cider, or pharmaceutically applicable products.

The term "metabolism" as used in the present context, relates to the products of metabolic functions in the biocatalyst. Metabolic products are usually obtained through the action of enzymes in the biocatalyst. When the biocatalyst is a living cell, where the term "living" refers to the biocatalysts ability to reproduce itself, the term "metabolism" relates to all metabolic products of such a living cell. The term "metabolism", however, also relates to a product or two or more different products produced by a single type of enzyme or other biocatalytically active species, which is not part of a "living" cell.

The term "biocatalytic conversion", as used in the present context, refers to the conversion of degradable carbohydrates into at least one gas and at least one product, as mentioned above, by a biocatalyst.

The term "biocatalyst", as used in the present context, refers to a microorganism or enzyme which is able to metabolically convert biocatalytically convertible material into at least one gas and at least one product. Examples for biocatalysts are microbes like bacteria, fungi or yeasts like Aspergillus niger, Rhizopus ozyzae, Zymomonas mobilis or Sacchcaromyces cerevisiae.

The term " aqueous solutions containing biocatalytically convertible material ", as used in the present context, refers to an aqueous solution of biocatalytically convertible material, which can be biocatalytically converted into at least one gas and at least one product by a biocatalyst as mentioned above. The term encompasses all types of such aqueous solutions, irrespective of their origin and their concentration of biocatalytically convertible material. Typical aqueous solutions like they are used in a preferred embodiment of the present invention, are wort, fruit juice, berry juice, sugar syrup, starch syrup, plant hydrolysates, sugar syrups flavoured by any fruit, berry, malt or similar extracts or any other aqueous substrate that is used in the production of any alcoholic beverage, especially beer, cider, wine or liquor. In a further preferred embodiment of the invention, the term stands for wort. The concentration of biocatalytically convertible matter in these examples can, of course, be varied widely, depending on how much alcohol and how much sweetness the end product is expected to have. A typical total concentration of biocatalytically convertible materials can range from 1 to about 400 g/l, e.g., 10 to about 300 g/l, or about 50 to about 250 g/l, including naturally present and added bioconvertible material.

The term "biocatalytically convertible", as used in the present context, stands for the property of a material to be converted into at least one gas and at least one product by a biocatalyst.

The term "converting/degassing stage", as used in the present context, refers to a unit comprising at least one bioreactor, where biocatalytically convertible material is biocatalytically converted by at least one biocatalyst, irrespective of shape, volume, model or other features of the bioreactor, and at least one degassing device, where gases produced during bioconversion can be removed.

A converting/degassing stage can solely consist of one bioreactor and one degassing device, it can, however, also comprise additional other devices, e.g., one or more devices for the removal of solids, which can be added between the bioreactor and the degassing device of after the degassing device, or both.

The term "bioreactor", as used in the present context, refers to a space, where a biocatalytical conversion can take place. The term "bioreactor" comprises any device allowing the contact of a solution to be biocatalytically converted with a biocatalyst, like a stirred tank reactor, a fluidized bed reactor, a basket reactor, a plug flow reactor or a filter reactor, like a membrane filter reactor or a ceramics filter reactor. It is also possible to use a combination of two or more of the mentioned reactor types as a bioreactor. A single vessel containing a high number of defined flow paths where biocatalytic conversion can take place, is still defined as one bioreactor, regardless of the number of defined flowpaths or biocatalytic conversion stages it encloses. An example for such a vessel having a high number of flowpaths is a column reactor, having a ceramic insert or a number of ceramic inserts forming channels, where a liquid flow is essentially or completely directed through the channels of the insert or inserts.

Bioreactors, as defined in the present context, generally have at least one inlet and at least one outlet. An aqueous solution to be biocatalytically converted is fed to the bioreactor through at least one inlet, a biocatalytically converted product is withdrawn through at least one outlet. A bioreactor can have more than one inlet and more than one outlet. Different inlets can be used to supply the bioreactor with feed at different places in the reactor, e.g., to obtain a more uniform performance of the bioreactor or to supply the bioreactor with different feeds in different places. The bioreactor can also have more than one outlet, e.g., to withdraw products at different stages of bioconversion.

In a basic set-up of the present invention, a converting/degassing stage contains only one bioreactor, having at least one inlet and at least one outlet. It is, however, also possible, to use two or more bioreactors in one converting/degassing stage. If two or more bioreactors are used, they can be interconnected serially or in parallel.

The term "serial interconnection", as used in the context of the present invention, refers to a series (line) of two or more devices, i.e., bioreactors, after the other, where the effluent of a first device is used as the input for a second device. The effluent of a the second device can be used as the input of a third device, etc., up to a last device in the series (line).

The term "parallel interconnection", as used in the context of the present invention, refers to a series of two or more devices parallel to each other, having a common feed line and a common effluent line or outlet.

A serial interconnection of two or more bioreactors can be useful, when different aims are to be achieved in each of the bioreactors. If two or more bioreactors are used in serial interconnection, not all of the bioreactors have to contribute to the gas formation. In some instances it might, e.g., be preferred, to achieve the production of certain products in a first bioreactor, before a second product is produced in a second bioreactor. For instance, in the production of alcoholic beverages, a first bioreactor can be used to achieve flavour formation, a second bioreactor can be used for the production of alcohol. If two or more stages of flavour or alcohol formation are needed or preferred, the number of bioreactors increases.

In a preferred embodiment of the invention, the maximum number of serially interconnected bioreactors is about 50, depending on the size of the bioreactors and the expected product output.

It is also within the scope of the present invention that two or more bioreactors are interconnected in parallel. Parallel interconnection means that the bioreactors have a common feeding line for each inlet of the bioreactors, supplying each of the bioreactors with an identical feed of an aqueous solution containing biocatalytically convertible material, or two or more common feeding lines, when the bioreactors have more than one different inlets and each of the different inlets is supplied with a different feed.

A parallel interconnection of bioreactors can, e.g., be useful, if a high product output is desired and the size of the bioreactors to be used is limited, e.g., due to technical or economical reasons. If two or more bioreactors are interconnected in parallel, it is preferred that each of the bioreactors performs the same task. In the production of alcoholic beverages, serially interconnected bioreactors can, e.g., perform the production of flavour compounds or production of alcohol.

The number of bioreactors interconnected in parallel can be very high, depending on the size of the employed bioreactors and the desired throughput. An economical upper limit for the number of bioreactors in parallel interconnection is about 10.000. Generally, the number of bioreactors in parallel interconnection will be less than about 1000 or less than about 500, e.g., about 100 or 50 or less.

It is also within the scope of the present invention that two or more bioreactors can be serially connected to form a line and two or more of such lines are connected in parallel. As an example for the production of alcoholic beverages, a line can consist of a first bioreactor, producing a certain flavour of the beverage and a second bioreactor, producing alcohol. Two or more of such lines can then be interconnected in parallel, each input of the first bioreactors in the line being fed from a common feeding line and output of the second bioreactors in line supplying a common line for product withdrawal. Such a system can of course be scaled up to a higher number of serially connected bioreactors forming a line and a high number of such lines being interconnected in parallel.

Additionally, one bioreactor or two or more of the bioreactors in each converting/degassing stage can have means, for reversing the flow direction in the bioreactor without changing the flow direction in the converting/degassing stage. This can be especially useful in a plug flow reactor, where upon reversal of the flow direction a fluidized bed reactor results.

Summarising, a converting/degassing stage can have one or more bioreactors, where two or more bioreactors can be interconnected serially or in parallel. It is within the scope of the present invention that other types of connections between two or more bioreactors can be used, e.g. to obtain a more uniform flow, to reduce pressure drops, or the like.

A converting/degassing stage according to the present invention, also comprises at least one degassing device.

The term "degassing device", as used in the present context, comprises any device capable of removing gases from liquids, like any stripping device, a gas permeable membrane unit, a hydrocyclone or a flash tank, where gas is removed from a gas loaded liquid by increasing the surface area of the liquid. A degassing device according to the present invention has at least one inlet for a product stream to be degassed and at least one outlet for a degassed product stream. In a preferred embodiment, the degassing device also has at least one outlet for removed gas. A "degassing device" according to the present invention can operate at any pressure above, at or below ambient pressure (about 1 bar), as long as it performs the task of removing a gas or a mixture of two or more gases from a liquid, either partially or completely.

In the present invention, the degassing device, or the two or more degassing devices, is (are) connected to the bioreactor or the two or more bioreactors in such a way that the effluent (product stream) from the outlet of the bioreactor or the outlets of the two or more bioreactors is fed to the inlet of the degassing device or the two or more degassing devices. In one converting/degassing stage, at least one outlet of a single bioreactor or at least one outlet of each of the two or more bioreactors is connected to the inlet of one degassing device or the inlets of two or more degassing devices.

When two or more degassing devices are used, they can be in serial or parallel interconnection. In a preferred embodiment of the invention, the two or more degassing devices are in parallel interconnection.

The degassing devices can have means to allow an operation of the device at reduced pressure. Generally, an operation under reduced pressure is achieved by applying a vacuum at the device, usually through one or more vacuum pumps connected to the device by one or more outlets located above the liquid level in the device. The pressure in the degassing device can be from about 0,01 bar up to slightly below the pressure in the bioreactors, e.g., from about 0,1 bar to about 10 bar or from about 0,2 bar to about 4 bar, as long as the task of removing at least part of the gas is achieved.

The degassing devices, as described in the present context, can have means to limit or prevent foaming in the degassing device. Such means can be mechanical means like ultrasound emitters or water spray or can be means to allow for the addition of chemical defoaming agents, like silicon based defoaming agents as they are known in the art.

Summarizing, a converting/degassing stage can have one or more degassing devices, where two or more degassing devices can be interconnected serially or in parallel. It is within the scope of the present invention that other types of connections between two or more degassing devices can be used.

A converting/degassing stage, according to the present invention, can, apart from the devices described above, comprise additional devices to improve the operability of the entire stage and the method according to the invention. Such additional devices (unit operations) can be heat exchange devices, devices for the removal of solids, valves, valve-control units, devices for gas injection, e.g., injection of air, devices for the addition of biocatalysts, devices for the withdrawal of liquid or the addition of liquid, and the like.

The additional devices can be positioned anywhere in the described converting/degassing stage, e.g., between two or more bioreactors or between two or more degassing devices or between the bioreactor or the two or more bioreactors and the degassing device or the two or more degassing devices.

In a preferred embodiment of the invention, a converting/degassing stage comprises at least one device for the removal of solids. If the converting/degassing stage comprises only one such device, it is preferred, to position it between the bioreactor and the degassing device or the two or more degassing devices, or, if the converting/degassing device comprises two or more bioreactors, between the last bioreactor in line or the last bioreactors of a parallel assembly of two or more bioreactors and the degassing device or the two or more degassing devices. If two or more devices for the removal of solids are employed, at least one of the devices is positioned as described above and the remaining devices are preferably positioned downstream from the bioreactors, preferably at or near at least one outlet of the bioreactors. In a further preferred embodiment of the present invention, if two or more bioreactors are used in serial interconnection, a device for the removal of solids is positioned between each of the bioreactors in line. It is within the scope of the invention to use any device which is suited to remove solids from a liquid stream as a device for removing solids according to the present invention. Examples for such devices are static or dynamic filters or centrifuges like a disc stack centrifuge. Other mechanical separation methods are applicable as well.

A converting/degassing stage can also comprise one or more heat exchange devices for heating or cooling the liquid stream. The term "heat exchange device", as used in the present context, relates to a device capable of heating or cooling a liquid or gas stream passing through the device. Such heat exchange devices can be positioned anywhere in the converting/degassing stage, wherever heating or cooling is needed, e.g. a heating device can be positioned upstream from the inlet of a degassing device or two or more degassing devices in order to heat the liquid entering the degassing device to facilitate gas removal from the liquid. Further heat exchange devices can be positioned downstream from one or more degassing devices in order to cool the liquid leaving the outlet of the degassing device or degassing devices.

The bioreactor or the bioreactors used in the present invention are loaded with a biocatalyst or two or more biocatalysts, as described above. If two or more biocatalysts are employed in one bioreactor, they can be employed as a mixture or separate, e.g., in sequential order, if the type of bioreactor and biocatalysts allow for a separate arrangement. The biocatalyst or the two or more biocatalysts can be used as a dispersion (fluid bed) in the liquid to be biocatalytically converted or they can be used in a fixed bed. The biocatalyst can be used in its free form or it can be chemically or mechanically fixed on a carrier material.

In a preferred embodiment of the present invention, the biocatalyst or the two or more biocatalysts are chemically or mechanically fixed on a carrier material. As a carrier material any solid material capable of chemically or mechanically immobilizing biocatalysts like the biocatalysts described above can be used. Examples for suitable carrier materials are glass beads, alginate beads, granular DEAE-cellulose or lignocellulose-material, like wood chips or nutshells, e.g., coconut shells. The carrier materials are loaded with the biocatalysts according to procedures known in the art. Wood chips are especially suited to mechanically immobilize biocatalysts. It is further possible to chemically alter the surface of the above mentioned carrier, if needed, to enable the material to chemically bind a biocatalyst or to improve its ability to do so. Chemical alterations of the above mentioned surfaces usually result in the ability of the material to exchange anions or cations, especially anions. Any process for the surface alteration of the above mentioned materials in the above mentioned way can be used. An especially suited process is described in the German patent application DE 198 48 623.5 which, especially its description of carrier materials, their preparation and their loading with biocatalysts, is incorporated into this text by reference.

Suitable processes utilizing glass beads are described in DE 41 11 879 and DE 41 37 474. Suitable glass beads are, e.g., obtainable through Schott Glaswerke, Mainz, Germany, e.g., the glass beads of the SIRAN®-type.

In a preferred embodiment of the invention granular DEAE-cellulose or wood chips or coconut shells are used as carriers. Suitable types of DEAE-cellulose, their manufacturing and loading (immobilization) processes and are described e.g. in US-A 5,001,063, DE-C 31 30 178, US-A 4,355,117,DE-C 28 15 908, US-A 4,110,164 or especially US-A 5,079,011, which are incorporated herein by reference.

It is within the scope of the invention, to either have only one converting/degassing stage or to have two or more converting/degassing stages in an apparatus according to the present invention. If two ore more converting/degassing stages are used, the respective stages can be interconnected serial or parallel, or, if the number of converting/degassing stages allows for, both modes of connection can be used. With regard to the modes of interconnection, the explanation above, regarding the interconnection of bioreactors or degassing devices, applies also here. If two or more converting/degassing stages are interconnected in a serial manner, the maximum number of serially connected stages is only limited by space, technical or economical considerations, the theoretical number can be very high. A practical number can be about 100 or less, e.g., 50, 20, 10 or 5 converting/degassing stages. The number is, of course, also determined by the desired throughput of the system

If parallel interconnection is chosen as a mode for operating the converting/degassing stages, the number of parallel stages is, theoretically, unlimited. Practical considerations applied to the maximum number of parallel stages are identical to the considerations for parallel bioreactors, as explained above.

An apparatus according to the present invention thus comprises at least one converting/degassing stage consisting of at least one bioreactor and at least one degassing device located downstream from the bioreactor or the bioreactors. The most basic set-up of an apparatus according to the present invention in which the method of the present invention can be performed, comprises at least one circulating device, e.g., a pump, a bioreactor, loaded with at least one biocatalyst, a degassing device, a circuit line, having an inlet connected with at least one outlet of at least one degassing device, and at least one outlet connected with at least one inlet of at least one bioreactor via a device for circulating the aqueous solution, at least one feeding line connected to the circuit line at a location downstream of the device for circulating the aqueous solution, and at least one line for product removal, connected to the circuit line at a location upstream of the circulating device.

The apparatus according to the present invention comprises at least one feeding line to supply the apparatus with fresh feed, i.e., fresh aqueous solution containing the material to be biocatalytically converted. The number of feeding lines can, of course, be higher than one, different lines carrying different or identical feeds. If different feeds are used in different feeding lines, they can be mixed to an essentially uniform solution before entering the apparatus or at least before entering the first bioreactor or bioreactors, as explained below. One or more of the feeding lines can also be used for cleaning operations of the apparatus, a feeding line can, e.g., be connected to a storage tank holding a cleaning liquid used to clean the apparatus after a certain period of operation.

The flow direction in normal mode of operation in the apparatus according to the present invention, is chosen so that the fresh aqueous solution is first directed through a bioreactor or two or more bioreactors in parallel interconnection. Such a bioreactor or such two or more bioreactors are referred to as a "first bioreactor" or "first bioreactors". The converting/degassing stages this bioreactor or these bioreactors belong to, are referred to as "first converting/degassing stage" or "first converting/degassing stages", if the apparatus according to the present invention has two or more converting/degassing stages in serial interconnection.

The degassing device or two or more degassing devices in parallel interconnection which the aqueous solution leaves before at least part of the converted and at least partially degassed solution is mixed with fresh aqueous solution from the feeding line, is referred to as "last degassing device" or "last degassing devices". The converting/degassing stages which this degassing device or these degassing devices belong to, are referred to as "last converting/degassing stage" or "last converting/degassing stages", if the apparatus according to the present invention has two or more converting/degassing stages in serial interconnection.

The circuit line is used to connect the last degassing device or the last degassing devices with the first bioreactor or the first bioreactors, to form a reaction loop.

The apparatus according to the present invention has at least one circulating device, e.g., a pump. The circulating device can be located anywhere within the reaction loop, e.g., within a converting/degassing stage or in the circuit line. In a preferred embodiment of the invention, the circulating device is located within the circuit line. If two or more circulating devices are used, they can be located anywhere along the circuit line or within one or more converting degassing stages.

The apparatus according to the present invention comprises at least one outlet, providing for the withdrawal of product or products. Additional outlets (withdrawal lines) can, e.g., be located within one or more converting/degassing stages, e.g., after a bioreactor or after a degassing device in order to obtain samples representing different stages of a process in the reaction loop or even final products, which differ from the products obtained after the last degassing device or degassing devices.

The apparatus according to the present invention can also comprise one or more buffer tanks, preferably located after the last converting/degassing stage or the last converting/degassing stages. A buffer tank can be used to maintain a constant level of liquids in the reaction loop.

The apparatus according to the present invention can further comprise one or more holding tanks for storage of the aqueous solution in the apparatus, e.g., in cases of maintenance or cleaning of the reactor, where the reactor has to be partially or completely emptied.

The apparatus according to the present invention can also comprise components like valves, devices or inlets for adding a gas, e.g. oxygen, or two or more gases to one or more of the devices in the reaction loop, devices for controlling product parameters like density, gravity, pH, gas content, ethanol content, pressure, temperature and the like.

In the method according to the invention, an aqueous solution containing biocatalytically convertible material is fed to a reaction loop, having one or more converting/degassing stages in parallel or serial interconnection
In a first process step the aqueous solution containing biocatalytically convertible material is fed in continuous flow to the inlet of a first bioreactor or first bioreactors, loaded with at least one biocatalyst, where it is contacted with the at least one biocatalyst under formation of a biocatalytically converted solution containing at least one gas and at least one product. Depending on the apparatus used to perform the method according to the invention, the converted solution leaving the outlet of the first bioreactor or the first bioreactors can be fed to a second bioreactor or second bioreactors, and eventually further bioreactors in serial connection, depending on the configuration of the apparatus, as explained above.

In a preferred embodiment of the present invention, at least one bioreactor is loaded with a biocatalyst which is immobilized on a carrier.

In a second process step, the biocatalytically converted solution is transferred from an outlet of a last bioreactor or last bioreactors to an inlet of a degassing device or degassing devices, depending on the configuration of the apparatus, as explained above, where it is at least partially degassed in a continuous flow to form a converted, degassed solution.

As already explained above, the converted and degassed solution can be fed to a second and eventually further converting/degassing stage, depending on the configuration of the apparatus the method is performed with.

In a further preferred embodiment of the present invention, the converted solution leaving the bioreactor or the two or more bioreactors is continuously treated to remove insoluble solids before entering the degassing device. This can be especially advantageous, when using a biocatalyst which is not immobilized on a carrier or when an immobilized living population of biocatalyst, e.g. yeast cells, in the bioreactor is increasing and biocatalyst cells are produced, which are not fixed on the carrier anymore. The above mentioned biocatalysts can leave the bioreactor and may cause fouling and clogging of downstream devices. If the method is performed employing two or more converting/degassing stages and a solids removal step is desired, the step of removing the solids from the converted solution can be performed in all, several or only one of the converting/degassing stages.

The biocatalytically converted and possibly solid-stripped solution from a bioreactor or two or more bioreactors is directed to a degassing device or two or more degassing devices, where gas formed during the passage of the bioreactor or bioreactors is removed from the solution. The removal of gas can be performed under any pressure which allows for the removal of a desired amount of gas from the solution. It is possible to remove the gas while the degassing device is under pressure or at ambient pressure (about 1 bar): It is, however, also possible to remove the gas at reduced pressure in the degassing device, i.e., at a pressure of below 1 bar, e.g. 0.5 bar or less.

When leaving the degassing device, the product has left a converting/degassing stage, unless the degassed product is subjected to further processing steps like heat exchange, pasteurisation or filtering.

The degassed product can then be transferred into a successive further converting/degassing stage, which is serially connected to the outlet of the prior converting/degassing stage. In case the converting/degassing stage the product has just passed is the last converting/degassing stage as explained above, a part of the biocatalytically converted and degassed product is withdrawn from the product stream after leaving the last converting/degassing stage as a final product. The remaining product is circulated back to the first converting/degassing stage in line, or to a common feeding line feeding the inlets of a row of parallel converting/degassing stages, via a circuit line into a first bioreactor or first bioreactors, together with fresh feed, being introduced through a feed inlet, as described above.

During or after each converting/degassing stage or after a number of successive or parallel converting/degassing stages, the product can be subjected to a heat exchanging step in order to keep the temperature at a constant level. In such a heat exchange step the temperature can be adjusted in order to ensure optimum conditions for the metabolism of the biocatalyst. Usually the heat exchange step will be necessary in order to dissipate the heat of reaction generated in the bioreactors.

The method according to the invention is usually performed under a continuous flow, but can also be performed under non-steady flow conditions like pulse-flow. The term "continuous flow", as used in the present context, refers to a flow which is continuous for a certain period of time, e.g., an hour, a day, a week, or more. The flow does not have to be steady, but the rate of flow can vary according to process conditions.

In a preferred embodiment of the present invention, the method is used for the production of ethanol, especially for the production of alcoholic beverages like beer, wine, cider or liquors, especially beer.

The circulation flow rate of the aqueous substrate within the reaction loop is an important parameter, since the production capacity for the product, e.g. ethanol, is dependent on the flow rate. It has also been shown that, when using a biocatalyst which is immobilized on a carrier, the flow rate affects the number of biocatalyst cells which remain bound in the carrier. With high flow rates, the biocatalyst outflow is increased, consequently product formation may also be dependent on the amount of biocatalyst bound in an immobilized system, and not only on the throughput rate of the system. If the flow rate is too high, the product formation is incomplete and/or inadequate. By controlling the flow rate, the product concentration can be controlled. Exemplary for ethanol formation, typically a concentration which is from about 0.05 % to about 20 % by volume relative to the total volume of the aqueous substrate (solution) is desired. By adjusting the flow rate, the loop reactor can yield an ethanol concentration within this range. The lower limit can generally be influenced by the rate of gas removal or the adjustment of the value of feed-rate (rate of feed supply to the reaction loop) versus the circulation flow rate.

The bioreactor can be pressurized. Usually operating pressures of between 1 and 10 bar are applied. Generally, the operating pressure is chosen so that the carbon dioxide formed in one pass will stay in solution at the operating pressure, in order to avoid gas evolution in the bioreactor. In a preferred embodiment the pressure is chosen to be between about 1.1 and 4 bar, e.g. between 1,5 and 3,5 bar.

The process temperature is generally chosen to be between a lower temperature, where the biocatalyst has just low metabolic activity and an upper temperature, where the biocatalyst is just not denaturated and still active. A lower limit for the process temperature is about 0°C, an upper limit is about 40°C. In a preferred embodiment the process temperature is adjusted to be between about 10 and about 25°C.

In the case of the production of ethanol under the formation of carbon dioxide, the recycle rate is chosen such that the carbon dioxide formed in one pass will stay in solution at the operating pressure.

In a further preferred embodiment of the invention, the flow direction in one or more bioreactors of each converting/degassing stage is reversed intermittently for a short period of time, e.g. 5 minutes, or longer, e.g. 5 hours. This procedure is especially useful for bioreactors of the plug flow type. The flow direction in the bioreactors can be reversed in short cycles, e.g. every hour or less, or in longer cycles, e.g. every 12 hours, every day, every week or even more, e.g. every month or every two months. The reversal of flow direction can prevent channel formation in the carrier bed and pressure build-up in the reactor and has shown to improve the overall performance of the method.

The method according to the invention can also comprise a cleaning step, where the apparatus the method is performed with is cleaned in a usual manner.

The method according to the present invention can further comprise additional steps, improving the overall performance or allowing to achieve or maintain a certain standard of the products produced by the process. Especially in the production of alcoholic beverages it can be advantageous to submit the feed entering the loop reactor to preparational measures like filtering, heating, cooling, pasteurizing or to a chemical or biocatalytical preconversion of some or all contents of the feed.

In a further preferred embodiment of the invention, chemical and/or physical parameters of the liquid entering the reaction loop and of the product withdrawn from the reaction loop are measured periodically or continuously. Such measurements can be used to control the overall performance of the reaction loop by adjusting the feed rate and the circular flow rate accordingly. A continuous measurement allows for steady concentration of biocatalytically convertible material in the reaction loop and for a constant product quality.

The loading of the reaction loop is generally performed according to standard loading procedures for bioreactors. In the production of alcoholic beverages with immobilized yeast cells, a bioreactor or the bioreactors is loaded with the respective carrier or the respective carriers and the whole reaction loop is cleaned with a cleaning liquid, e.g., a sodium hydroxide solution. The cleaning liquid is circulated for about 1 to about 100 hours in the reaction loop. Following the cleaning step, the reaction loop is usually flushed with water and eventually the contents of the reaction loop can be neutralized or acidified to a pH of about 1 to about 8 with an acidic solution. Inoculation and propagation of the bioreactors with yeast can be achieved according to standard procedures, e.g., as described in the examples below.

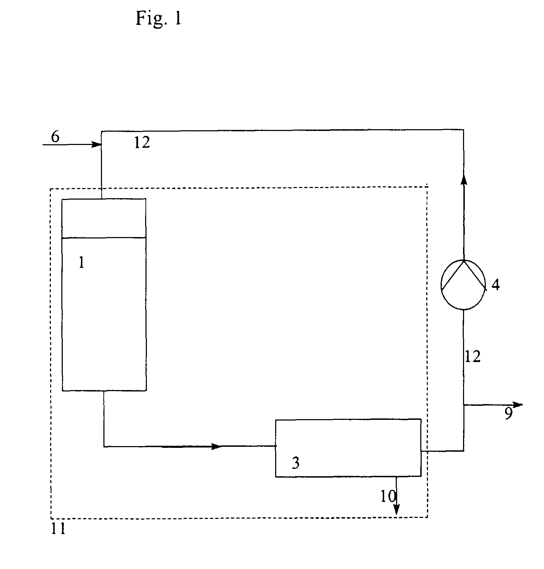

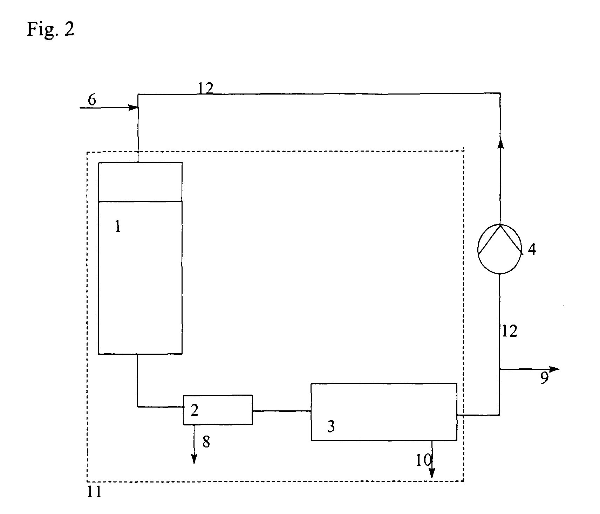

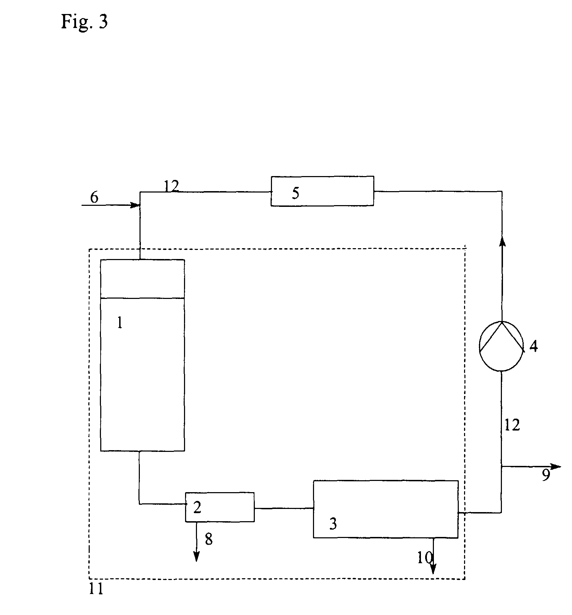

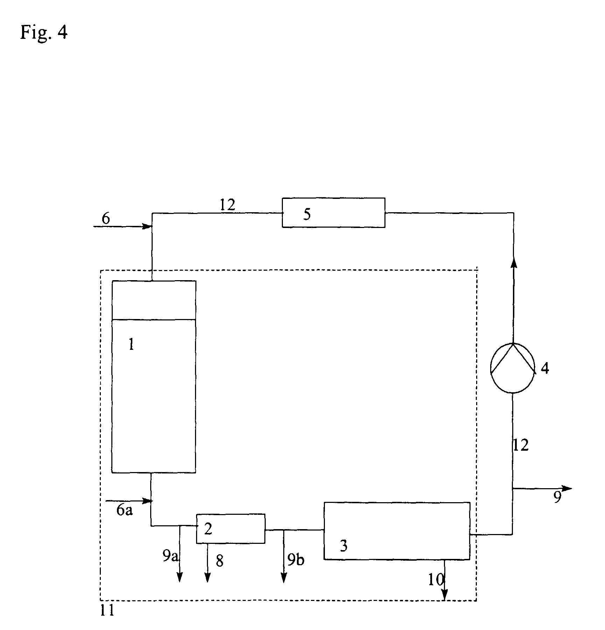

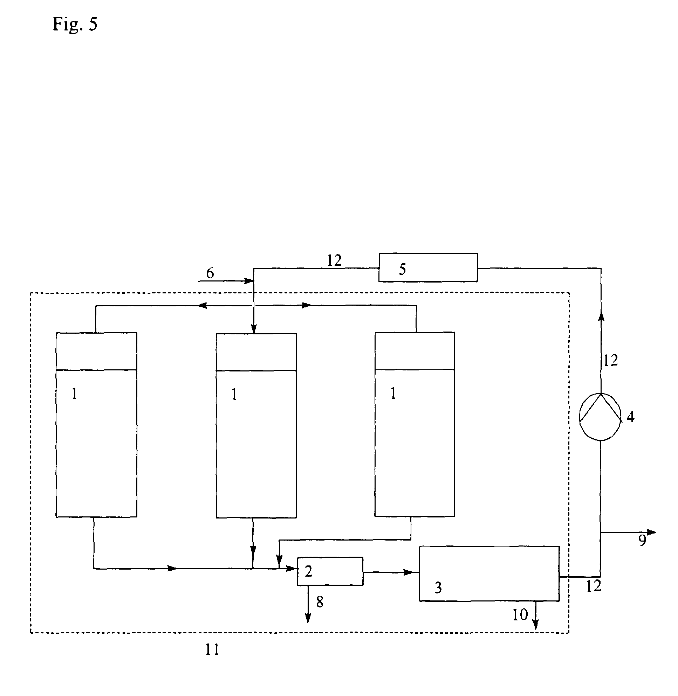

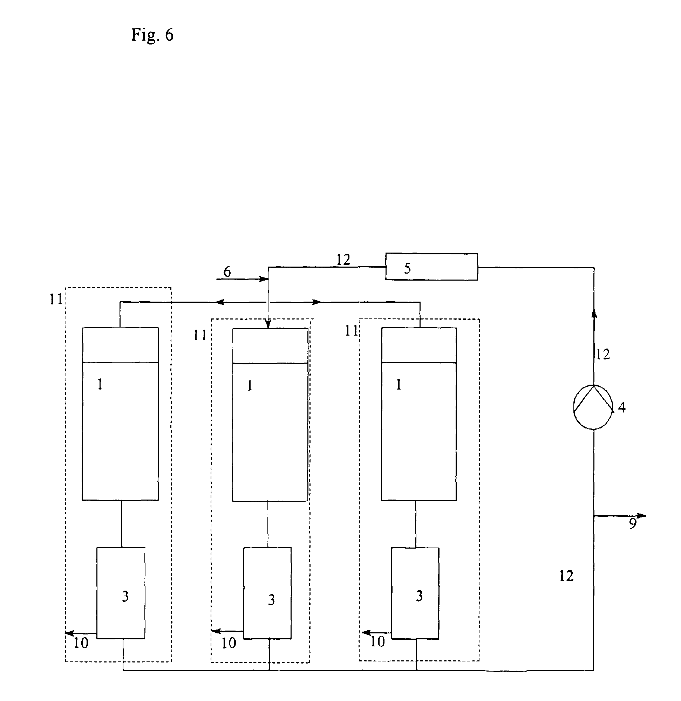

A simplified process diagram using an apparatus according to the present invention is presented in the drawing, consisting of Figures 1 to 6.

Figure 1 shows a reaction loop reactor with one converting/degassing stage (11). Fresh feed of an aqueous solution of biocatalytically convertible materials is introduced through a feed line (6) into a circuit line (12), carrying a stream of biocatalytically converted, degassed product. The mixture of fresh feed and biocatalytically converted, degassed product flows into a bioreactor (1), containing at least one biocatalyst, where it is at least partially biocatalytically converted to at least one gas and at least one product. The biocatalytically converted product flows into a degassing device (3), having a gas outlet (10) where the gas or the gasses contained in the biocatalytically converted product are at least partially removed. When leaving the degassing device, the biocatalytically converted, degassed product leaves the converting/degassing stage (11) through the circuit line (12). Part of the biocatalytically converted, degassed product is withdrawn at an outlet (9) located downstream from the degassing device. The remaining biocatalytically converted, degassed product is fed by a circulation device (4) through the circuit line (9) back to the converting/degassing stage (11), where it is mixed with fresh feed from the feed line (6) and the mixture is introduced into the bioreactor (1) of the converting degassing stage (11).

Fig. 2 shows the reaction loop of Fig.1 having a device for the separation of solids (2) between the bioreactor (1) and the degassing device (3). The device for the separation of solids (2) has an outlet (8) for the removed solids.

Fig. 3 shows the reaction loop of Fig. 2, having a heat exchanging device (5) between the circulating device (4) and the bioreactor (1).

Figure 4 shows the reaction loop of figure 3, having an additional feed line (6a) and two additional outlets (withdrawal lines) (9a, 9b) for withdrawal of aqueous solution in different process stages.

Figure 5 shows a reaction loop having a converting/degassing stage (11) with three bioreactors (1) in parallel interconnection , a device for the removal of solids (29 and a degassing device (3) with a gas outlet (10).

Figure 6 shows a reaction loop having three converting/degassing stages (11) in parallel interconnection, each converting/degassing stage (11) having a bioreactor (1) and a degassing device (3) with a gas outlet (10).

It is apparent from the drawings, that further reaction loops according to the invention can be constructed by applying the teaching of the text above.

In a preferred embodiment of the invention, the converting/degassing stage of the apparatus according to the invention comprises a device for removing solids (2), having an outlet (8) for removed solids, between the bioreactor and the degassing device. In a further preferred embodiment, the apparatus comprises a heat exchanger (5). The inventive apparatus can comprise one or more converting/degassing stages in serial or parallel fluid interconnection. In a preferred embodiment, the number of converting/degassing stages is 1 or 2.

A further subject of the invention is a method for the continuous biocatalytic conversion of aqueous solutions containing biocatalytically convertible material, characterized in that an apparatus as described above is used.

A further object of the present invention is the use of an apparatus as described above, for the bioconversion of an aqueous solution containing biocatalytically convertible material under the formation of at least one gas and at least one product.

In addition to what has been described above, the apparatus may contain auxiliary equipment needed for the practical operation of the process, like buffer and storage tanks, a wort filter, a pasteurizer, a tank for the preparation of cleaning liquids and a tank for the storage of beer during cleaning of the reactor.

In its simplest form, an apparatus according to the invention has only one reactor connected to the degassing device. In different applications, however, the apparatus may comprise several reactors combined in series or in parallel, and only a part of these may be within the loop. This allows for flexibility in designing processes having distinct reaction phases. For example, a beverage fermentation process may have an aerobic and an anaerobic phase. The aerobic phase is executed outside the loop in a separate reactor whose feed is aerated. The product of the aerobic phase, from which the oxygen has been consumed, is then directed to the anaerobic loop where the actual ethanol biocatalytic conversion takes place.

Practical execution of a process according to the present invention is described in detail in the following examples, yet without restricting the applicability of the invention in other processes. The essential feature of the invention is the circulation, which makes it possible to keep the gas in solution within the reactor and remove it outside the reactor. Hence the invention may be applied in any biocatalytic conversion or other process involving formation of carbon dioxide or other gaseous components in the solution to be processed.

### Example 1. Loading and cleaning of the reactor

Forty kilograms of granular DEAE-cellulose prepared commercially according to US-A 4,355,117 was charged in a column reactor with an inner diameter of 40 cm and a height of 120 cm to form a carrier bed of 100 litres settled volume. 120 litres of a 4 % solution of sodium hydroxide was prepared in a tank having a total volume of 150 litres. The tank temperature was set at 85 °C and the solution was pumped from the tank upflow through the reactor at a flow rate of 200 litres/hour with the outflow directed back to the tank. After 18 hours of circulation the tank was drained and flushed and filled with hot process water (temperature not controlled). The outflow from the reactor was directed to drain and hot water was pumped through at a flow rate of 400 litres/hour until the pH of the reactor outflow had dropped below 10 units. A solution of 1 kg of citric acid in 120 litres of cold process water (temperature not controlled) was prepared in the tank. The outflow from the reactor was directed back to the tank and the acid solution was circulated through the reactor at a flow rate of 400 litres/hour for 2 hours. The tank was drained and flushed and filled with cold water. The outflow from the reactor was directed to drain and cold water was pumped from the tank through the reactor for another 2 hours.

### Example 2. Inoculation of the reactor

The tank was drained of water and filled with wort having an original gravity of 14,5 ° P (degrees on the Plato scale) from an industrial brewery. The outflow from the reactor was directed to drain and wort was pumped from the tank upflow to the reactor at a flow rate of 200 litres/hour keeping the wort volume in the tank at about 100 litres. Pumping was continued until the outflow had changed from water to wort. Oxygen was sparged through the tank to saturate the wort. 20 litres of harvested yeast from an industrial fermentation plant was pitched into the tank to make a slurry of a volume of 120 litres containing about 4 x 10⁸ cells/ml. A lid was placed on the tank to prevent contamination and to keep foaming in control. The outflow from the reactor was directed back to the tank and the yeast slurry was circulated upflow through the reactor at a flow rate of 200 litres/hour.

### Example 3. Colonization of yeast and pre-fermentation of wort in the circulation loop

Pressure in the reactor was set at 2,0 bar and the circulation temperature at 20 °C. Circulation was continued upflow through the reactor with intermittent oxygen sparging. The carbon dioxide formed in the fermentation was released in the tank. The resulting foam settled to form a liquid layer in the bottom of the tank allowing continuous pumping. After two days' pre-fermentation the circulation was stopped. The tank was drained and flushed with water. 120 litres of a 2 % solution of sodium hydroxide was prepared in the tank. The tank temperature was set at 85 °C and the solution was circulated through the pipes bypassing the reactor. After 2 hours the circulation was stopped. The tank was drained and flushed and filled with cold water, which was pumped through the pipes for 1 hour.

### Example 4. Continuous fermentation with fresh feed

The tank was drained and filled with ready fermented green beer from an industrial fermentation plant. Circulation was started downflow through the reactor at a flow rate of 200 litres/hour. The pressure setting was kept at 2,0 bar and the temperature at 20 °C. Circulation was directed through a disc-stack centrifugal separator to remove yeast shedding from the reactor and through a heat exchanger for pasteurization. Fresh wort having an original gravity of 14,5 °P was fed into the circulation loop at a constant flow rate of 6,0 litres/hour. A pump maintaining a constant level in the tank was used for product withdrawal. After ten days' circulation the original gravity of the feed wort was increased to 18 °P and the feed flow rate was reduced to 5,0 litres/hour. The residual fermentable sugar content in the effluent green beer was about 0,75 °P. The beer was sampled daily for the analysis of volatile aroma components. The analysis results at one week's intervals are presented in Table 1 below.

**Table 1.**

| Flavour analysis of the effluent beer, concentration in parts per million | | | | |
|---|---|---|---|---|
| Day no. | 8 | 15 | 22 | 29 |
| | | | | |
| Acetaldehyde | 6 | 7 | 7 | 7 |
| Acetone | 1,0 | 0,6 | 1,0 | 1,0 |
| Ethyl acetate | 26 | 23 | 32 | 35 |
| Isobutyl acetate | <0,5 | <0,5 | <0,5 | <0,5 |
| Ethyl butyrate | 0,15 | 0,15 | <0,05 | 0,10 |
| Propanol | 37 | 15 | 30 | 29 |
| Isobutanol | 21 | 13 | 22 | 20 |
| Isoamyl acetate | 0,9 | 0,9 | 0,8 | 1,2 |
| 2-Methylbutanol | 11 | 8 | 13 | 12 |
| 3-Methylbutanol | 64 | 36 | 62 | 51 |
| Diacetyl | 0,18 | 0,35 | 0,32 | 0,32 |
| 2,3-Pentanedione | 0,02 | 0,03 | 0,05 | 0,05 |

After three weeks of circulation the reactor top pressure started to rise indicating build-up of yeast in the reactor. During the following two weeks the pressure rose from 2,2 to 3,9 bar and the circulation was stopped after a total of 37 days of production.

### Example 5. Rejuvenation of the yeast loading and subsequent continuous fermentation

The tank was drained and filled with fresh wort, which was oxygenated. Flow mode was changed to upflow and circulation was continued at 200 litres/hour. Yeast was released from the reactor to build up a concentration of 10⁸ cells/ml in the circulating beer. After 18 hours the circulation was stopped and the tank was drained and cleaned as described in Example 3 and filled with ready fermented green beer. Circulation was started downflow at 200 litres/hour with a feed of fresh wort having an original gravity of 15,0 °P at 6,0 litres/hour. The reactor pressure was restored to 2,0 bar and remained at that during the following two weeks, after which the circulation was stopped.

### Example 6. Scale-up of continuous fermentation

Based on the experiences gained from the experiments described in Examples 1 to 5, a bigger pilot unit was constructed for the fermentation of beer using a scale-up factor of 10. The unit comprised two feed tanks for wort with pipe connections to an industrial brewhouse, a wort pasteurizer, a wort filter, a column reactor, a disc-stack separator, a flash tank for CO₂ removal, a buffer tank to control the liquid volume in the loop, a heat exchanger, a tank for preparation and circulation of cleaning liquids, a yeast propagator and necessary pumps. The equipment was piped and instrumented to allow for automatic operation of different cleaning, yeast propagation, inoculation and fermentation cycles. The production loop consisted of the reactor, the separator, the flash tank, the buffer tank and the heat exchanger in this order.

The reactor was loaded with 400 kilograms of granular DEAE-cellulose to form a bed of a volume of 1000 litres. The reactor and other equipment were cleaned as described in Example 1. 50 litres of harvested yeast from an industrial fermentation plant were transferred to a yeast propagator for the final propagation. The ready inoculum of 400 litres containing a total of 10⁸ cells/ml was pitched to the reactor and circulated upflow through the reactor for two hours. The loop was filled with oxygenated wort of an original gravity of 18 °P and circulation was started downflow at a flow rate of 2000 litres/hour. After two days fermentation the gravity had dropped close to full attenuation and the feed of fresh wort and the withdrawal of product were started at a flow rate of 50 litres/hour. The outlet pressure was set at 2,0 bar and the temperature at 24 °C. The product from the centrifuge entered the flash tank through a nozzle in the center of the tank forming an umbrella-shaped spray, which hit the tank walls. The falling liquid film was collected from the tank bottom without significant foaming.

During the first twelve days the reactor pressure increased steadily from 2,2 to 3,6 bar, after which wort feed and product withdrawal were stopped and the direction of circulation was changed to upflow. A thick yeast suspension released from the bed was directed to drain. The direction of circulation was changed back to downflow and the loop beer was oxygenated in circulation for an hour, after which normal operation with feed of fresh wort was resumed. The inlet pressure had dropped back to 2,5 bar.

The system worked steadily causing a gravity drop of 14 °P in a residence time of 20 hours. The circulating product before and after the reactor was sampled daily for the analysis of volatile aroma components. An analysis of both inlet and outlet of three days in succession is presented in the following Table 2.

**Table 2.**

| Flavour analysis of circulating beer before and after the reactor, concentrations in ppm | | | | | | |
|---|---|---|---|---|---|---|
| | Day 8 in | out | Day 9 in | out | Day 10 in | out |
| | | | | | | |
| Acetaldehyde | 6,4 | 6,2 | 6,4 | 6,7 | 7,3 | 7,1 |
| Acetone | 0,9 | 1,0 | 1,0 | 1,0 | 1,5 | 1,5 |
| Ethyl acetate | 26,3 | 27,9 | 26,5 | 27,4 | 27,9 | 28,5 |
| Isobutyl acetate | 0,05 | 0,10 | 0,05 | 0,05 | 0,05 | 0,05 |
| Ethyl butyrate | 0,20 | 0,25 | 0,15 | 0,20 | 0,15 | 0,20 |
| Propanol | 31,2 | 31,8 | 29,3 | 30,7 | 30,3 | 31,2 |
| Isobutanol | 28,0 | 29,0 | 28,5 | 29,7 | 29,7 | 31,3 |
| Isoamyl acetate | 0,37 | 0,38 | 0,39 | 0,41 | 0,25 | 0,25 |
| 2-Methylbutanol | 15,7 | 16,2 | 15,4 | 16,2 | 15,5 | 16,1 |
| 3-Methylbutanol | 57,8 | 60,4 | 56,0 | 59,2 | 55,8 | 58,4 |
| Diacetyl | 0,39 | 0,40 | 0,39 | 0,42 | 0,30 | 0,33 |
| 2,3-Pentanedione | 0,059 | 0,061 | 0,061 | 0,068 | 0,048 | 0,054 |

Samples were also collected for sensory flavour assessment. Because the product was unmaturated green beer, the samples were conditioned for tasting using a normal laboratory procedure. The product was tasted by an experienced panel and regarded to be good and typical of its kind. The flavour was, however, fruitier and smoother compared with a conventionally fermented control.

## Claims

1. A method for the continuous biocatalytic conversion of aqueous solutions containing biocatalytically convertible material in a reactor having one or more converting/degassing stages in parallel or serial interconnection, each converting/degassing stage comprising a bioreactor or two or more bioreactors and a degassing device or two or more degassing devices, where the bioreactor or the bioreactors and the degassing device or the degassing devices are in serial interconnection, and where in each converting/degassing stage in a first process step an aqueous solution containing biocatalytically convertible material is fed to the inlet of the bioreactor where it is contacted with a biocatalyst under formation of a biocatalytically converted solution containing at least one gas and at least one product, and where in a second process step the biocatalytically converted solution is transferred from an outlet of the bioreactor or a common outlet line of two or more bioreactors to an inlet of the degassing device or a common inlet line to two or more degassing devices, where it is at least partially degassed to form a degassed solution and at least part of the degassed solution leaving the outlet of the last degassing device or a common outlet of two or more last degassing devices in line, is fed to the inlet of the first bioreactor in line or to a common feeding line for the inlets of two or more first bioreactors, in a continuous flow.

2. Method according to claim 1, characterized in that the bioreactor is a stirred tank reactor or a column reactor or a fluidized bed reactor or a basket reactor or a plug flow reactor or a membrane filter reactor or a combination of two or more of the mentioned reactors.

3. Method according to claim 1 or 2, characterized in that the degassing device is a gas permeable membrane unit, a hydrocyclone or a flash tank or a combination of two or more of them.

4. Method according to one of claims 1 to 3, characterized in that the pressure at the inlet of the first bioreactor in line is 1,1 to 10 bar.

5. Method according to one of claims 1 to 4, characterized in that the converting/degassing stages are parallel interconnected.

6. Method according to one of claims 1 to 5, characterized in that the number of converting/degassing stages is 1 to 100

7. Method according to one of claims 1 to 6, characterized in that the biocatalytically converted solution is treated to remove insoluble solids from it before entering the degassing device.

8. Method according to one of claims 1 to 7, characterized in that the temperature of the solution is adjusted after one or more degassing stages in a heat exchanger.

9. Method according to one of claims claim 1 to 8, characterized in that the biocatalyst is immobilized on a carrier

10. Method according to one of claims 1 to 9, characterized in that the biocatalyst is yeast.

11. Method according to one of claims 1 to 10, characterized in that the aqueous solution containing biocatalytically convertible material is wort.

12. Method according to one of claims 1 to 11 characterized in that the solution is green beer.

13. Apparatus for the continuous biocatalytic conversion of aqueous solutions containing biocatalytically convertible material comprising:
- a bioreactor or two or more bioreactors loaded with at least one biocatalyst, each bioreactor having at least one inlet and at least one outlet,
- a circuit line having an entrance connected with at least one outlet of at least one bioreactor via at least one device for degassing the aqueous solution, and at least one exit connected with at least one inlet of at least one bioreactor via a device for circulating the aqueous solution,
- at least one feeding line connected to the circuit line at a location downstream of the device for circulating the aqueous solution, and
- at least one outlet (withdrawal line) connected to the circuit line at a location upstream of the device (4) for circulating the aqueous solution.

14. Apparatus according to claim 13 comprising at least two bioreactors (1) in a serial interconnection.

15. Apparatus according to claim 14 comprising at least two bioreactors (1) in a parallel interconnection.

16. Apparatus according to one of claims 13 to 15, characterized in that it comprises two or more converting/degassing stages.

17. Apparatus according to one of claims claim 13 to 16, characterized in that at least one bioreactor (1) is a stirred tank reactor, a fluidized bed reactor, a basket reactor, a plug flow reactor, or a membrane filter reactor.

18. Apparatus according to one of claims 13 to 17, characterized in that at least one degassing device (3) is a gas permeable unit, a hydrocyclone, or a flash tank.

19. Apparatus according to one of claims 13 to 18 further comprising at least one device (2) for removing solids, having a discharge line (8) for removed solids, positioned in said circuit line upstream or downstream of a bioreactor (1).

20. Apparatus according to claim 9, characterized in that at least one device (2) for removing solids comprises at least one centrifuge or filter.

21. Apparatus according to claim 20, characterized in that the device (2) for removing solids comprises a disc stack centrifuge.

22. Apparatus according to one of claims 13 to 21 further comprising at least one heat exchanger (5).

23. Apparatus according to claim 22, characterized in that the at least one heat exchanger (5) is positioned at a location downstream of the degassing device (3).

24. Apparatus according to claim 22, characterized in that the at least one heat exchanger (5) is positioned between the device (4) for circulating the aqueous solution and the feed line (6).
